# EUROPEAN PATENT APPLICATION

(11) **EP 4 751 714 A1**
(43) Date of publication of application: **03.06.2026**
(21) Application number: 24216367.3
(22) Date of filing: 29.11.2024
(51) Int. Cl.: A61K 8/73, A61K 9/00, A61K 47/36, A61L 27/36

(54) **COMPOSITION, DEVICE AND KIT FOR REGENERATIVE AESTHETICS**

(71) Applicant: Linio Biotech Ltd, 00150 Helsinki (FI)
(72) Inventor: Uusmies, Jertta-Riina, 00150 Helsinki (FI); Ylikomi, Timo, 00150 Helsinki (FI); Hautanen, Veera, 00150 Helsinki (FI); Kriel, Haydn, 00150 Helsinki (FI); Reijonsaari, Karita, 00150 Helsinki (FI); Vinkanharju, Antti, 00150 Helsinki (FI)
(74) Representative: Primrose Oy

(57) **Abstract**

The invention relates to a composition comprising a cell-free lipoaspirate-derived preparation comprising at least one of a lipid fraction and an aqueous fraction of a lipoaspirate and at least one biodegradable crosslinked hydrogel. The invention further relates to a device and a kit comprising the composition. The composition, the device and the kit can be generally applied for aesthetic treatment of soft-tissue loss or deficit.

## Description

### TECHNICAL FIELD

The invention belongs to the field of regenerative aesthetics, in particular to a composition, a device and a kit for use in volume augmentation and maintenance thereof.

### BACKGROUND

Patients, and their treating healthcare practitioners, are increasingly looking for more effective and minimally invasive approaches towards management of soft tissue loss, especially to compensate subcutaneous volume loss. Particularly in the facial area, volume augmentation with injectable products has been the state of the art with a clear move away from surgical invasive procedures such as surgical facelifts.

Volume augmentation products, also known as fillers, have recently been cast in a bad light due to the occurrences of unnatural appearance, overfilled face syndrome and risk of short- and long-term adverse effects. There is a trend toward more natural-looking filler treatments reflecting a growing consumer preference for subtle enhancements that maintain the integrity of one's unique features rather than create dramatic transformations. As patients increasingly seek aesthetic outcomes that are soft and realistic, practitioners are shifting away from traditional, high-volume fillers that can lead to overcorrection and visible signs of treatment. This has led to the development of innovative treatment techniques, patient specific treatment regimens and a desire for new products that offer natural and regenerative solutions. Products that could regenerate or restore natural tissues would ensure that a person can receive suitable treatment and still appear their natural selves.

This has emerged the field of regenerative aesthetics, which has already been growing exponentially because of advancements in biotechnology, stem cell research, and biomaterials, combined with an increasing demand for minimally invasive, natural-looking solutions for aging, injury repair, and cosmetic enhancement. These advancements pursue to develop more precise and effective approaches to healing, restoring volume, and improving skin quality, while leveraging the body's own regenerative capabilities.

Currently the aesthetic treatment options for tissue regeneration include both cell-containing and cell-free technologies. Cell-containing approaches include fat grafting (i.e., autologous fat transfer) and autologous stem cell treatments. In fat grafting, the fat is harvested from one area of the patient's body, typically through liposuction, purified and processed (sometimes to micro- or nanofat), and then injected into another bodily area of the same patient to restore soft tissue volume. While fat transfer is widely employed in aesthetic procedures, it presents several significant challenges. These include unpredictable fat resorption rates, expensive and labour intensive procedure and the inherent risks of the liposuction (e.g. anaesthesia and invasiveness). Studies have shown that the resorption rate of grafted fat can vary significantly, typically ranging from 20 % to 90 %, leading to inconsistent reductions in the volume initially implanted. This variability often necessitates additional procedures and can sometimes yield less desirable aesthetic results, making it difficult to achieve long-lasting outcomes with fat grafting. The main reason behind fat resorption is insufficient revascularisation of transferred tissue.

Cell-free technologies like decellularized adipose tissue extracellular matrix are safer than cell-containing technologies in terms of adverse immune reactions and allow allogenic applications. However, although decellularized adipose tissue matrix provides a scaffold for cells to attach, it lacks key biostimulatory signalling to actively promote tissue regeneration.

Biostimulatory agents (e.g. peptides and growth factors) have been found to have superficial effects on skin improvement, but they cannot provide substantial volume restoration, particularly in cases of significant subcutaneous fat loss. Moreover, single growth factor treatments may carry a potential risk of stimulating growth of abnormal cells, increasing the chance of developing cancer, especially in individuals who are already at risk.

Thus, there is a need for safe and effective biostimulatory cell-free technologies that offer natural-looking and long-lasting volumising effect through soft tissue regeneration.

### SUMMARY

In an aspect, the present invention provides a composition comprising: a cell-free lipoaspirate-derived preparation comprising at least one of a lipid fraction and an aqueous fraction of a lipoaspirate; and at least one biodegradable crosslinked hydrogel.

In another aspect, provided is a device for regenerative aesthetics, wherein the device comprises the composition according to any embodiment disclosed herein.

In a further aspect, provided is a kit for regenerative aesthetics, wherein the kit comprises the composition according to any embodiment disclosed herein.

In still further aspects, provided is use of the composition, the device or the kit according to any embodiment disclosed herein for cosmetically treating a human body, preferably a facial and/or neck area of a human body.

In an even further aspect, provided is a non-therapeutic method for cosmetically treating a human body, the method comprising the step of applying the composition, the device or the kit according to any embodiment disclosed herein to a human body, preferably to a facial and/or neck area of a human body.

Further aspects, embodiments and details are set forth in the following figures, detailed description, examples, and dependent claims.

### BRIEF DESCRIPTION OF THE DRAWINGS

The accompanying drawings illustrate several embodiments of the disclosed subject matter, and together with the description, serve to explain principles of the disclosed compositions and methods.

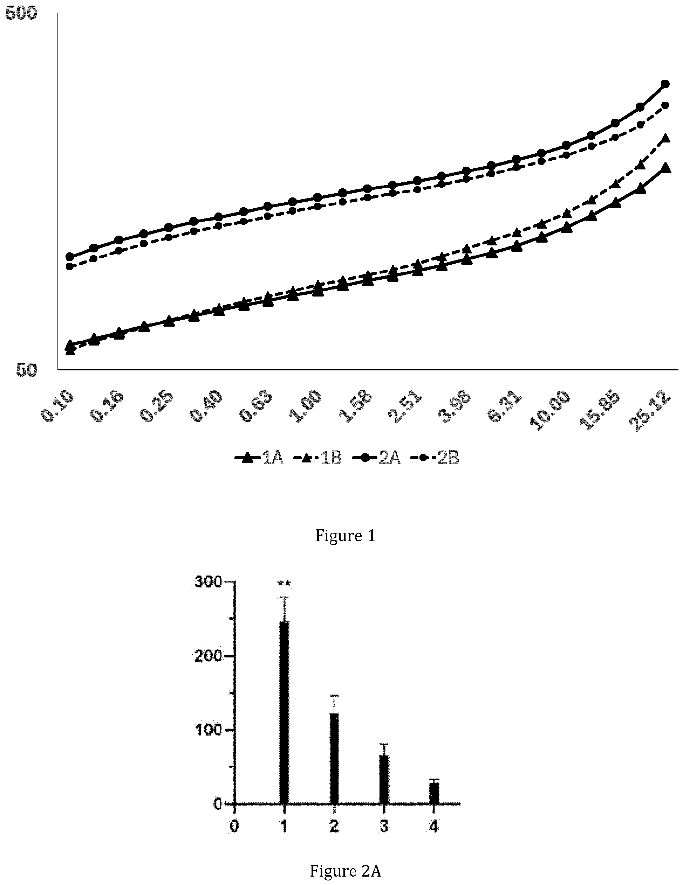

Figure 1 is a curve diagram showing the storage modulus (G') of two commercial hyaluronic acid hydrogel fillers mixed with ringer acetate solution as measured on the same day of preparation and measured after storage under different conditions i.e., storage modulus measured of Belotero^{®} Volume on the day of mixing (1A) and after storage for one week at room temperature (1B) and Juvéderm^{®} Voluma on the day of mixing (2A) and after storage for 1 day at +4 degrees Celsius (2B).

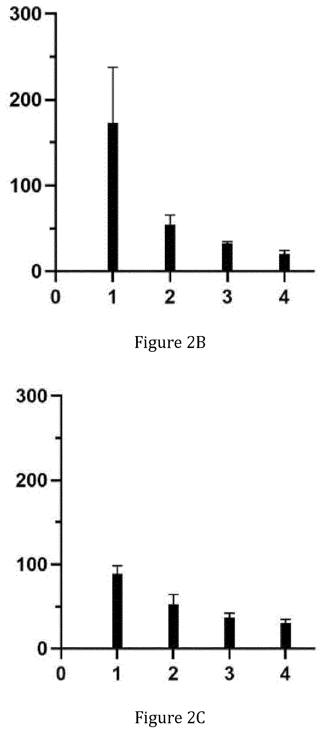

Figure 2A-2C are histograms showing a protein release profile for three different combination products during a 4-day inspection period. Figure 2A shows the results obtained with Restylane^{®} Lyft, Figure 2B shows the results obtained with Juvéderm^{®} Voluma, while Figure 2C shows the results obtained with Belotero^{®} Volume as the crosslinked hyaluronic acid (HA) component. Asterixses in Figure 2A indicate statistical significance (p-value < 0.01) in two sample t-test when comparing day one protein release between Restylane^{®} Lyft and Belotero^{®} Volume.

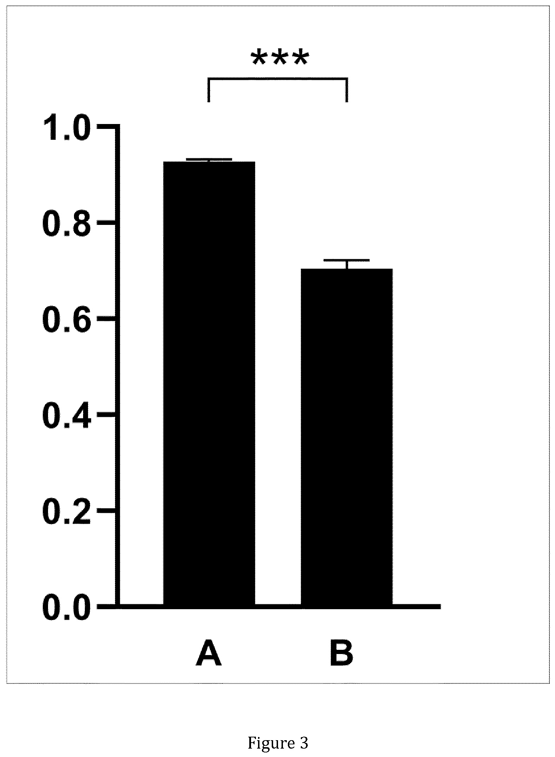

Figure 3 is a histogram illustrating fibroblast migration after a 24-hour induction with a combination product containing a cell-free lipoaspirate-derived preparation or a combination product containing ringer acetate (column A); p-value on two-sample t-test < 0.001. The y-axis represents the diameter of the cell-free area after the induction period and is thus inversely proportional to the speed of fibroblast migration.

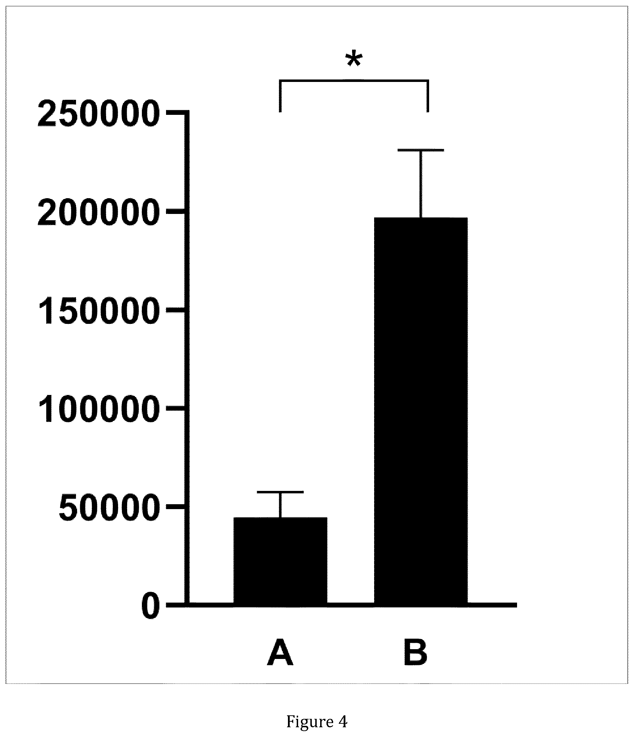

Figure 4 is a histogram illustrating intracellular triglyceride formation in human adipose stem cells after exposure to a combination product containing a cell-free lipoaspirate-derived preparation (column B) or a combination product containing ringer acetate as a vehicle control (column A); p-value on two-sample t-test < 0.05. Increased intracellular triglyceride formation indicate adipose stem cell development into mature adipocytes.

### DETAILED DESCRIPTION

The following detailed description illustrates aspects and embodiments of the present invention and ways in which they can be implemented. Although some modes of carrying out the present invention have been disclosed, it is to be understood that other embodiments for carrying out or practicing the present invention are also possible. In other words, the scope of the present invention will be limited only by the appended claims.

It is also to be understood that the terminology used herein has the same meaning as commonly understood by one of ordinary skill in the art to which this invention belongs, unless otherwise specified.

The present invention provides various compositions, devices and kits that may be applied, for instance, for aesthetic dermatology, especially for regenerative aesthetics. According to some embodiments, said compositions, devices and kits are intended for various skin regeneration purposes, as will be explained in more detail later in the detailed description. In some further embodiments, especially in those intended for subcutaneous administration, said compositions, devices and kits are intended for aesthetic treatment of soft tissue loss, more specifically for providing and maintaining volume augmentation.

In an aspect, provided is a composition which comprises or consists of a cell-free preparation comprising or consisting of a lipid fraction and/or an aqueous fraction of a lipoaspirate, and a biodegradable cross-linked hydrogel.

In some embodiments, the composition may comprise one or more additional components selected from the group including, but not limited to, aqueous media, buffers, pH modifiers, stabilizers, preservatives, cryoprotectants, salts, sugars, gelling agents, antioxidants, and/or local anaesthetics, but since none of the additional components is allowed to contain cells or be non-biodegradable (i.e. biostable), the composition as a whole is also cell-free and biodegradable. Moreover, all the components must be, and hence also the composition is, biocompatible.

As used herein, the term "cell-free" refers to an entity that is devoid or essentially devoid of cells. Techniques for determining whether a given preparation is cell-free or not are readily available in the art.

Being cell-free provides several advantages, including for example, low immunogenicity and hence reduced risk of adverse effects when administered to a human body. It follows that the composition of the invention may be provided as an allogenic product, i.e., as a product suitable for use by subjects different from the donor of the lipoaspirate. There are also many practical benefits when factors such as cell viability during storage or after administration need not be considered.

As used herein, the term "biodegradable" refers to a material that, when in contact with physiological fluids, starts to degrade but preserves its mechanical properties for a certain period of time, and is eventually safely absorbed and excreted by the body. In other words, biodegradable materials disappear over time and do not require surgical removal.

Biodegradability is crucial for the hydrogel to be used in the present composition because it ensures that the hydrogel provides temporary structural support, enables natural tissue regeneration, minimizes the risk of foreign body reactions, and facilitates the controlled release of bioactive agents. Ultimately, a biodegradable composition promotes a smoother healing process, avoiding long-term complications and aligning with the body's natural tissue repair mechanisms.

As used herein, the term "biocompatible" refers to a material that, upon administration *in vivo,* is compatible with living tissues and does not induce substantial undesirable side effects, such as toxicity reactions, significant irritation, or immune responses such as allergy. Biocompatible materials may, however, without compromising their biocompatibility, cause mild, transient inflammation which accompanies implantation of essentially all foreign material into a living organism and which is also associated with the normal healing response. In other words, the benefit/risk ratio must be reasonable. In essence, the term "biocompatible" means the same as "physiologically acceptable".

As used herein, the term "composition" refers to an entity in which its components are provided either as a mixture, preferably as an essentially homogeneous mixture, or separately as distinct components. If provided separately, the components are to be combined and mixed prior to administration to a human body. The resulting mixture may be denoted as a combination product to distinguish it from a composition in which the components are provided separately. Also, a composition in which its components are provided as a ready mixture may be denoted as a combination product. The combination product may be a cosmetic combination product. In an embodiment, the combination product may be denoted as a biostimulatory filler.

In another aspect, provided is a device for regenerative aesthetics, the device comprising the composition of the invention. To put it differently, provided is a device for regenerative aesthetics, wherein the device comprises a cell-free preparation comprising or consisting of a lipid fraction and/or an aqueous fraction of a lipoaspirate, and a biodegradable cross-linked hydrogel.

In a further aspect, provided is a kit for regenerative aesthetics, the kit comprising the composition of the invention. To put it differently, provided is a kit for regenerative aesthetics, wherein kit comprises a cell-free preparation comprising or consisting of a lipid fraction and/or an aqueous fraction of a lipoaspirate, and a biodegradable cross-linked hydrogel.

As used herein, the term "lipoaspirate" refers to material obtainable by liposuction. It may originate from a single human subject, or it may be a mixture of lipoaspirates originating from more than one human subject. Both options are included in the term "a lipoaspirate".

Liposuction is a surgical removal of subcutaneous adipose tissue (i.e., hypodermal adipose tissue) by means of aspiration cannulas introduced through small skin incisions, assisted by suction. Various liposuction techniques are readily available and have evolved as technology has improved. Technological advancements added to the traditional dry liposuction technique include ultrasonic liposuction and radiofrequency-assisted liposuction to name some non-limiting examples. The current state-of-the-art liposuction technique is water-assisted liposuction, a technique that utilizes a pressurized stream of saline/aqueous buffered solution in an amount that is about two to three times larger than the volume of subcutaneous fat to be removed, thereby causing the fatty subcutaneous tissue layer to swell and become firm prior to aspiration and to ease the removal of subcutaneous fat/tissue from different areas in the body. Commonly used saline/aqueous buffered solution in water-assisted liposuction is called "tumescent solution". Accordingly, the liposuction technique can be called tumescent liposuction. Traditional tumescent solution contains local anaesthetics such as lidocaine and adrenaline or epinephrine. Notably, the present invention is not limited to any particular liposuction technique, as the choice of the technique often depends on patient characteristics and surgeon preference.

As used herein, the term "liposuction solution" refers to any physiologically acceptable aqueous solution suitable for use as an infiltrate in any liposuction technique, including not only the conventional tumescent liposuction but also other water-assisted liposuction techniques. Non-limiting examples of suitable liposuction solutions include 0.9 % sodium chloride (saline), Ringer Acetate, Ringerfundin^{®}, Ringer lactate and Normofundin^{®}. Those skilled in the art can select an appropriate aqueous buffered solution for each case. In some embodiments, the liposuction solution may contain any approved local anaesthetic, such as lidocaine or prilocaine, typically 0.5 % or 1 % lidocaine (up to 55 mg/kg). In some other embodiments, the liposuction solution may contain adrenaline/epinephrine, typically 1 % adrenaline (up to 7 mg/kg), for temporary vasoconstriction to prevent bleeding during liposuction. In some embodiments, the liposuction solution may contain both lidocaine (and/or any other appropriate local anaesthetic) and adrenaline, preferably in amounts mentioned above. Such a liposuction solution is traditionally called tumescent solution, although any buffered aqueous solution used in liposuctions is sometimes called as "tumescent solution", regardless of whether lidocaine or adrenaline/epinephrine is present or not. Therefore, the terms "liposuction solution" and "tumescent solution" as used herein are interchangeable, unless otherwise indicated. It follows that in some further embodiments, a liposuction liquid containing neither lidocaine (or any other local anaesthetic) nor adrenaline/epinephrine may still be called as "tumescent solution".

The term "spent liposuction solution" refers to a liposuction solution that has been used in liposuction, i.e. that has been in contact with human subcutaneous fat *in vivo* and/or after aspiration *ex vivo.* The spent liposuction solution comprises various at least partly water-soluble substances originating from the subcutaneous fat tissue.

When a lipoaspirate obtained by a liposuction technique that involves use of a liposuction solution is centrifuged or allowed to stand for a while, the lipoaspirate separates into different layers, namely into a lipid layer on the top (a first layer), an adipose tissue layer comprising adipocytes and other cells of the adipose tissue in the middle (a second layer), and an aqueous layer at the bottom (a third layer). The aqueous layer contains the spent liposuction solution used for the liposuction. Any cell or tissue debris contained in the lipoaspirate may separate into a fourth layer below the aqueous layer. However, as used herein, the term "bottom layer" refers to the third layer, and the term "middle layer" refers to the second layer as described above. In this context, the term "layer" can be used interchangeably with the term "fraction".

In accordance with the above, the term "aqueous fraction of a lipoaspirate" refers to the bottom fraction of a lipoaspirate or the bottom fraction of a spent liposuction solution, which bottom fractions (i.e. third fractions) form when said lipoaspirate or said spent liposuction solution that has been allowed to settle, i.e. separate into layers described above.

In accordance with the above, the term "lipid fraction of a lipoaspirate" refers to the top fraction of a lipoaspirate or the top fraction of a spent liposuction solution, which top fractions (i.e. first fractions) form when said lipoaspirate or said spent liposuction solution that has been allowed to settle, i.e. separate into layers described above.

As used herein, the term "fraction" is not limited to the whole fraction (i.e., layer) in question, but includes also any portions thereof. In other words, the present cell-free preparation need not contain the whole lipid fraction and/or the whole aqueous fraction of a lipoaspirate. To put it differently, cell-free preparations containing only a portion of the lipid fraction and/or a portion of the fraction of a lipoaspirate are encompassed by the cell-free lipoaspirate-derived preparation (CFLP) comprising at least one of a lipid fraction and an aqueous fraction of a lipoaspirate.

In some embodiments, liposuction solution used in the liposuction but not aspirated together with the adipose tissue (i.e., is collected to a separate container during or right after the liposuction) may be provided as the cell-free liposuction-derived preparation or used as an aqueous component thereof, for example by way of being added to the collected aqueous fraction of a lipoaspirate to increase its volume. In some further embodiments, any physiologically acceptable aqueous buffered solution/saline may be added to the lipoaspirate to increase the volume of the aqueous fraction, if desired.

In some embodiments, the spent liposuction solution, either as a collected aqueous fraction of a lipoaspirate or as a spent liposuction solution not forming part of a lipoaspirate, may be diluted or concentrated as desired by adjusting its volume using physiologically acceptable diluents and/or techniques readily available in the art.

In some embodiments, the cell-free lipoaspirate-derived preparation consists of a lipid fraction of a lipoaspirate. In some embodiments, the preparation comprises a lipid fraction of a lipoaspirate as the only lipoaspirate-derived material.

In some embodiments, the cell-free lipoaspirate-derived preparation consists of an aqueous fraction of a lipoaspirate and/or of a spent liposuction solution not being part of a lipoaspirate. In some embodiments, the preparation of the invention comprises an aqueous fraction of a lipoaspirate and/or a spent liposuction solution not collected as part of the lipoaspirate, as the only liposuction-derived material in the preparation.

In some embodiments, the cell-free lipoaspirate-derived preparation consists of a lipid fraction and an aqueous fraction of a lipoaspirate with or without being supplemented with a spent liposuction solution not being part of the lipoaspirate. In some embodiments, the preparation comprises a lipid fraction and an aqueous fraction of a lipoaspirate with or without being supplemented with a spent liposuction solution not collected as part of the lipoaspirate, as the only liposuction-derived material in the preparation. The lipid fraction may be the whole lipid fraction of the lipoaspirate, or a portion thereof.

In some embodiments, the cell-free liposuction-derived preparation comprises or consists of a spent liposuction solution without the adipose tissue fraction, i.e. without the middle fraction that forms when a lipoaspirate or a spent liposuction solution is allowed to settle. In some further embodiments, the preparation comprises a spent liposuction solution without the adipose tissue fraction, i.e. without the middle fraction that forms when a lipoaspirate or a spent liposuction solution is allowed to settle, as the only liposuction-derived material in the preparation.

In any of the above-described embodiments, the cell-free liposuction-derived preparation may have been supplemented with a spent liposuction solution which is not part of the actual lipoaspirate.

The cell-free lipoaspirate-derived preparation for use in the present invention is obtainable by a method in which a lipoaspirate is processed in a manner that results in a preparation that contains a lipid fraction and/or an aqueous fraction of the lipoaspirate, and is devoid of cells naturally present in an adipose tissue. This may be achieved in different ways. In some embodiments, the method comprises the steps of removing the adipose tissue fraction of a lipoaspirate, collecting the remaining lipid fraction and/or aqueous fraction, and sterile filtering the collected fraction(s). In some other embodiments, the adipose tissue fraction is not actively removed but only the desired fractions, i.e., either the lipid fraction, the aqueous fraction, or both are collected and subsequently sterile-filtered. Since no washing steps are carried out prior to collecting the aqueous fraction, it contains the spent liposuction solution. It follows, that the collected fraction may or may not contain a local anaesthetic, such as lidocaine, and/or adrenaline. It also follows that the lipid fraction is not removed by any washing steps. In some embodiments, the lipoaspirate is shaken gently and/or allowed to settle such that it separates into different layers prior to removing the adipose tissue fraction and/or collecting the lipid and/or the aqueous fraction. The adipose tissue fraction can be removed by any suitable technique available in the art. Also collecting of the lipid fraction and/or the aqueous fraction can be carried out by any suitable technique available in the art. Sterile filtering is usually carried out by using a 0.22 µm filter, i.e. a membrane filter having a pore size of 0.22 µm. Alternatively, sterile filtering may be carried out using a 0.2 µm filter, i.e. a membrane filter having a pore size of 0.2 µm.

It is to be understood that filtering of the remaining or collected fractions limits the particle size of various constituents in the resulting cell-free preparation, depending on the pore size of the filter used. If a 0.2 µm or a 0.22 µm filter is used, the preparation of the invention does not contain large intact extracellular matrix (ECM) proteins having at least one dimension that is larger than 0.2 µm or 0.22 µm, respectively.

In some embodiments, it may be beneficial to alter the composition of the cell-free preparation by fractionation or enriching it to increase the concentration of some component or alternatively removing or diminishing the concentration of some component.

In some embodiments it may be beneficial to add bioactive agents, including but not limited to growth factors, cytokines, growth factors, cytokines, lipids, ceramides, vitamins, to the product to boost the effects of the product.

Being cell-free means that the lipoaspirate-derived component of the present composition, combination product, device and kit is fundamentally different from any adipose tissue-derived stromal vascular fraction (SVF) which contains heterogeneous cell populations such as mesenchymal progenitor/stem cells, preadipocytes, endothelial cells, pericytes, T cells, and M2 macrophages. The SVF is obtainable from the adipose tissue fraction of a lipoaspirate. Accordingly, the component is also fundamentally different from nanofat, which is an emulsified and filtered adipose tissue fraction of a lipoaspirate, as well as from any cell-free extracts obtained from nanofat by removal of the cells. In other words, nanofat is obtained by discarding the lipid and the aqueous fractions of a lipoaspirate, and by emulsifying the remaining adipose tissue fraction, i.e. the cell fraction. It is to be understood that even if the emulsified cell fraction is processed further by removal of the cellular components, which process may involve use of an aqueous solution, the resulting cell-free extract does not qualify as the aqueous fraction of a lipoaspirate for use in the present invention, even if the nanofat-derived cell-free extract was aqueous.

Moreover, the cell-free lipoaspirate-derived component of the present composition, device and kit is fundamentally different from any decellularized fat-derived compositions available in the art. Such decellularized compositions are natural scaffolds derived from adipose tissue, in which the cellular and nuclear contents are eliminated, but the three-dimensional structure and composition of ECM are preserved.

In accordance with what is already indicated above the other main component of the present composition, combination product, device and kit is a biodegradable crosslinked hydrogel. The hydrogel component provides immediate volume augmentation after administration *in vivo.*

As used herein, the term "crosslinked hydrogel" refers to a hydrophilic, three-dimensional crosslinked polymer system capable of imbibing large amounts of water or biological fluids between their polymeric chains to form aqueous semi-solid or solid gel networks. Crosslinked hydrogels are primarily composed of water, which makes them biocompatible and suitable for mimicking the natural tissue environment. This allows cells to thrive in or around the hydrogel after administration, making them ideal for tissue engineering. Crosslinked hydrogels can be easily injected into irregular or hard-to-reach areas of the body, where they will conform to the space, making them excellent for applications like filling tissue defects and excellent for minimally invasive procedures.

Crosslinked hydrogels have a three-dimensional (3D) network formed by linking polymer chains either physically (through non-covalent interactions) or chemically (via covalent bonds). This crosslinking provides mechanical strength and structural stability, which is essential for mimicking the mechanical properties of native soft tissues (e.g., muscle, skin, cartilage). The degradation rate of hydrogels can be tuned and controlled through selection of the crosslinking technique to be used and particulars thereof, such as those affecting the degree and/or efficiency of crosslinking. In soft tissue engineering, the hydrogel should degrade at a rate that is compatible with the rate of new tissue formation. Uncrosslinked hydrogels lack this network and may degrade too quickly or in an unpredictable manner, compromising their ability to support cell growth, differentiation, and tissue formation over time.

Moreover, formulation of hydrogels can be adapted for compliance with a targeted indication and/or a desired release profile of lipoaspirate-derived bioactive substances (i.e., bioactive agents) contained in the hydrogel for optimal tissue repair and regeneration. These adaptations, recognized by those skilled in the art, can significantly influence the physicochemical properties of the hydrogel. Key adaptations may include, without limitation, choice of the crosslinking agent and adjusting the degree and efficiency of crosslinking. Additionally, the choice of liquid medium used during crosslinking, such as saline, phosphate buffer, Ringer's acetate, can also play a role. Format modifications, including the use of a sieve to create uniform gel particles and the decision to produce monophasic or biphasic formulations, are essential considerations. Furthermore, other manufacturing processes, such as cutting the gel into smaller pieces, dialysis or purification, filling containers, and steam sterilization, are relevant processing variables that may affect the final properties of the hydrogel. Each of these factors, and also specific variables that can be adjusted for a specific outcome, is well understood by those with expertise in the art. Hydrogels can also be specifically designed to have a sol-gel transition (i.e., they can exist in a liquid state before administration and then gel in response to physiological conditions such as body temperature or pH). This enables minimally invasive administration through a syringe or a cannula, offering benefits over more invasive surgeries required for implanting preformed scaffolds.

Depending on the choice of hydrogel the protein release rate may vary. In an embodiment, the hydrogel for use in the present invention provides a first-order protein release over four weeks after implantation. In this context, the protein release refers to the release of bioactive proteins of the lipoaspirate-derived preparation from the hydrogel component. In an embodiment, the hydrogel for use in the present invention provides a first-order protein release over three weeks after implantation. In an embodiment, the hydrogel for use in the present invention provides a first-order protein release over two weeks after implantation. In an embodiment, the hydrogel for use in the present invention provides a first-order protein release over one week after implantation. In an embodiment, the hydrogel for use in the present invention provides a first-order protein release over three days after implantation. In the above-mentioned embodiments, the protein release rates refer to those determined in protein release assays *in vitro.* Such assays are available in the art and are known to skilled persons.

Another preferred feature for the hydrogel to be used in the present invention is its ability to facilitate uniform mixing to achieve a homogeneous mixture with the lipoaspirate-derived component while minimizing deformations of the hydrogel. The hydrogel should retain its physicochemical properties, to an appropriate degree for the desired indication, even after repeated mixing, ideally achieving a homogenous mixture with as few cycles as possible. In an embodiment, the number of mixing cycles is 50-30 cycles, preferably 30-20 cycles, more preferably 20-10 cycles, and most preferably 10-5 cycles of back-and-forth transfer between syringes, cartridges, or similar containers under shear stress. Under ideal conditions homogenous mixing is achieved with as few cycles as possible while maintaining the desired physiochemical properties of the hydrogel for the indication in question.

Another preferred feature for the hydrogel is that after mixing with the lipoaspirate-derived component to form the combination product, that the mixture should preferably allow aspirating. Aspirating of crosslinked hydrogels is the process of pulling back on the plunger of syringe used for administration, whilst the needle or cannula is stationary and in situ, where filler is to be placed. This theoretically ensures the needle is not in a blood vessel and vulnerable to blockage by the subsequently injected filler. In general, monophasic hydrogels that are smoother and more uniform in structure are more amenable to aspiration compared to particulate biphasic hydrogels (large gel particles can obstruct the needle or cannula).

Preferred hydrogels for use in the present invention include hyaluronic acid-based hydrogels. As used herein, the term "hyaluronic acid" (HA), also known as "hyaluronan" or "hyaluronate" refers to a naturally occurring glycosaminoglycan commonly used in dermal fillers to reduce wrinkles and add volume to tissues. It is an anionic, non-sulphated glycosaminoglycan found abundantly in connective, epithelial, and neural tissues. HA exists in polymeric form, with a molecular weight that can reach several million Daltons. The human body typically contains around 15 grams of hyaluronan, approximately one-third of which is broken down daily by natural enzymes and free radicals within a few hours or days. This degraded HA is continuously replenished by new synthesis in the body.

Crosslinked hyaluronic acid is a modified form of HA created by chemically or physically linking its polymer chains to enhance its stability and longevity. In its natural state, HA breaks down quickly in the body due to enzymatic activity and free radicals. Crosslinking prevents this rapid degradation by forming a more resilient, three-dimensional network that can retain its structure for a longer period. This makes crosslinked HA ideal for use in the present composition and combination product, as it provides extended durability and better volumising effects compared to non-crosslinked HA. The degree of crosslinking can be adjusted to create products with varying consistencies and lifespans, making it possible to adjust the present composition and combination product for different aesthetic applications, such as filling of superficial defects such as wrinkles and volume augmentation of deeper volume deficit, including volumisation of cheeks and contouring of the chin to name few non-limiting examples.

The art is replete with different HA crosslinking technologies, including technologies based on chemical crosslinking (by permanent reaction) creating covalent bonds between the individual HA polymer chains, as well as technologies based on physical crosslinking (by reversible reaction) through forces such as hydrophobic, electrostatic, and hydrogen bonding between the individual HA polymer chains. Together these different technologies offer a multitude of opportunities for customization of the crosslinked hyaluronic acid with respect to its properties, such as storage modulus, cohesiveness, and longevity, and to meet diverse aesthetic needs. Those skilled in the art are able to select a suitable crosslinking technique depending on the desired properties of the crosslinked hyaluronic acid.

For example, physicochemical properties of crosslinked hyaluronic acid may be adjusted by one or more adaptations including: adjusting the degree and efficiency of crosslinking. of hyaluronic acid with a crosslinking agent, such 1,4-butanediol diglycidyl ether (BDDE) which is the most commonly used crosslinker, selecting the molecular weight of hyaluronic acid polymers prior to crosslinking, blending different molecular weights of hyaluronic acid polymer in a specific ratio, mixing with uncrosslinked hyaluronic acid (to make a product more injectable) and modifying the concentration of hyaluronic acid before the crosslinking process.

In the context of HA fillers used for wrinkle filling and volumisation, the terms storage modulus, cohesiveness, and longevity may be understood to be mean the following:
- The storage modulus (G'), measures the material's elasticity or firmness. It indicates the filler's ability to resist deformation and maintain shape under mechanical stress, with higher G' values representing firmer, more structured hyaluronic acid hydrogels suitable for volumising and lifting applications. This is typically measured from in the linear viscoelastic range of the frequency sweep in oscillatory rheometric analyses.
- Cohesiveness refers to the internal binding strength of an HA filler, or how well the gel particles stick together. High cohesiveness ensures that the filler maintains its integrity, resists fragmentation after injection, and stays in place for uniform volume distribution and natural tissue integration. This can be measured in several ways including drop weight, compression, tack, and dispersion time tests.
- Longevity describes the duration of filling effect of an HA after injection, determined by factors such as crosslinking density, resistance to enzymatic degradation, and how long the filler retains its structure in the tissue. Fillers with higher longevity provide longer-lasting aesthetic results before requiring re-treatment. This property can be tested *in vitro* degradation assays and also *in vivo* in real physiological conditions.

When defining HA product types it is useful to categorise crosslinked hyaluronic acid products into non-limiting generic descriptors (such as monophasic and biphasic, or cohesive and dispersive, or some hybrids of these) and, if relevant, to define these alongside proprietary names that are closely associated with specific manufacturing processes or specific product features to help clarify their different properties and applications.

Monophasic HA refers to a uniform, homogenous gel structure where the HA molecules are evenly distributed throughout the product. Monophasic gels are typically cohesive and have smooth consistency, offering better injectability and durability. These gels tend to flow more evenly, which results in a more natural aesthetic outcome, especially in dermal fillers. These are typically not passed through sizing screens/sieves and are not considered particulate or granular.

In contrast, biphasic HA products consist of two distinct phases: one phase of crosslinked HA particles suspended within a non-crosslinked HA matrix. This results in a more textured or granular gel with distinct particles, leading to a greater ability to mould and shape the gel once injected. The particles are typically consistent in size and are achieved in a downstream process of passing the gel through sizing screens/sieves. Biphasic gels are typically more granular and may be suitable for specific applications that require structural support and volume augmentation. In some cases, HA products may employ a mixture of both cohesive and dispersive properties or even combine monophasic and biphasic elements. These hybrid systems aim to balance the benefits of both technologies, offering the ease of injection and uniformity of monophasic gels along with the flexibility and moulding capability of biphasic gels. For example, a hybrid system might incorporate fine HA particles within a smooth, cohesive matrix, offering both volumising and contouring benefits.

Non-limiting examples of technologies available in the art for chemical crosslinking of HA include CPM^{™}, Hylacross^{™}, Vycross^{™}, NASHA^{™}, OBT^{™} or XpresHAn^{™}, and PNT^{™}. These represent the major technologies from HA filler products commercially available from the biggest brands.

CPM^{™} (Cohesive Polydensified Matrix, in Belotero products such as Belotero^{®} Volume and Belotero^{®} Intense by Merz Aesthetics) is a technology for providing a cohesive monophasic crosslinked hyaluronic acid gel with different densities of HA, creating a combination of high and low-density zones. Denser areas provide the volumising effect and areas of lesser density for cohesivity of the matrix. This type of hydrogel is typically achieved by a two-step crosslinking process (polydensified). CPM^{™} technology allows for smooth integration into the skin and the flexibility of the gel provides for a natural feel upon injection to adapt seamlessly to facial dynamics.

Hylacross^{™} (in Juvéderm products such as Juvéderm^{®} Ultra Plus 3 by Allergan) is a technology where the HA used for crosslinking is predominantly high-molecular weight HA. The cross-linked HA produced from this technology creates monophasic gels.

Vycross^{™} (in Juvéderm products such as Juvéderm^{®} Voluma and Juvéderm^{®} Volux by Allergan) is a technology for a providing monophasic crosslinked hyaluronic acid gel achieved by crosslinking blends of high- and low-molecular-weight HA to produce a smooth and more efficiently cross-linked gel.

NASHA^{™} (Non-Animal Stabilized Hyaluronic Acid, in Restylane products such as Restylane^{®} and Restylane^{®} Lyft by Galderma) is a technology for providing biphasic hydrogels consisting of a mixture of particles (of controlled sizes) of minimally crosslinked HA mixed with non-crosslinked HA (this is extractable added to facilitate injection). This provides for gels that have a firm texture and a more pronounced lifting effect.

OBT^{™} (Optimal Balance Technology) or XpresHAn^{™} is used to create Restylane products such as Restylane^{®} Defyne and Restylane^{®} Volyme by Galderma) and is a technology that produces flexible HA fillers where the firmness (or specifically storage modulus) is varied by applying different degrees of crosslinking.

PNT^{™} (Preserved Network Technology, in Teosyal products such as Teosyal^{®} RHA4 from Teoxane) is a technology for providing a monophasic crosslinked hyaluronic acid gel that is prepared using low temperature crosslinking to preserve long (high molecular weight) HA polymer chains that in turn require low amounts of crosslinker to achieve clinically desirable mechanical properties and durability. These less rigidly crosslinked HA chains are presumed to allow implants to better adapt to mechanical deformations such as muscle movements driving dynamic facial motion.

In an embodiment, the hydrogel component of the present composition, combination product, device or kit is monophasic HA-based hydrogel, such as monophasic HA. In another embodiment, the hydrogel component of the present composition, combination product, device or kit is cohesive HA-based hydrogel, such as cohesive HA. In yet another embodiment, the hydrogel component of the present composition, combination product, device or kit is monophasic and cohesive HA-based hydrogel, such as monophasic and cohesive HA.

Other hydrogels suitable for use in the present invention include crosslinked protein-based hydrogels, such as those based on collagen, gelatin, fibrin, silk fibroin, and/or elastin. Suitable further hydrogels include crosslinked, polyethylene glycol (PEG)-based hydrogels, and crosslinked, polysaccharide-based hydrogels, such as those based on agar/agarose, chitosan, and/or alginate. Suitable still further hydrogels include crosslinked peptide hydrogels, which may include short chain peptides and/or self-assembling hydrogels. Suitable still further hydrogels include crosslinked thermosensitive hydrogels (e.g., Chitosan/β-Glycerophosphate (C/GP) Hydrogel or similar that are liquid near room temperature and undergoes sol-gel transition on injection at body temperature +37 degrees Celsius.

Different biodegradable crosslinked hydrogels may also be used in any appropriate combination depending on the desired properties of the present composition, as is known to those skilled in the art.

As already mentioned, an aspect of the present invention provides a device and a kit comprising the present composition. The lipoaspirate-derived component and the hydrogel component of the composition may be provided either in combination, i.e. as a mixture, or separately in the device and in the kit. If provided separately, they are to be combined and mixed prior to administration to a human body. The resulting mixture may be denoted as a combination product to distinguish it from a composition in which the lipoaspirate-derived component and the hydrogel component are provided separately. Also, a composition in which its components are provided as a ready mixture may be denoted as a combination product.

As also already mentioned, the composition, and thus also the combination product, may contain one or more components additional to the lipoaspirate-derived component and the hydrogel component, including but not limited to, those selected from the group consisting of aqueous media, buffers, pH modifiers, stabilizers, preservatives, cryoprotectants, salts, sugars, gelling agents, antioxidants, and/or local anaesthetics. If the lipoaspirate-derived component and the hydrogel component are provided separately, the additional components may be provided together with either one or both of the lipoaspirate derived component and the hydrogel component. Those skilled in the art can easily select suitable additional components for each case.

Notably, the lipoaspirate-derived component may contain local anaesthetics, such as lidocaine or any other anaesthetic of the amino amide type with or without adrenaline or epinephrine, as part of the aqueous fraction of the lipoaspirate in accordance with what is disclosed above. Alternatively or additionally, local anaesthetics may be added into the present composition, more specifically either into the lipoaspirate-derived component or into the hydrogel component, or into both. Those skilled in the art know which local anaesthetic and dosage to choose depending on the particulars of each case.

Irrespective of whether or not the composition or the combination product includes one or more additional components set forth above, and irrespective of whether the various components forming the composition and the combination product are provided separately or in combination, administration into a human body requires that the combination product is sterile. Preferably, the lipoaspirate-derived preparation is sterilised by filtering, whereas a preferred way of sterilizing the hydrogel component is steam sterilisation.

In accordance with what is disclosed above, an embodiment provides a device in which the cell-free lipoaspirate-derived preparation and the at least one biodegradable crosslinked hydrogel are provided as a premixed composition in a single container, such as a vial, an ampoule, a bottle, a syringe, a cartridge or the like. The premixed composition may be in aqueous form, e.g. as a semisolid gel, as a solid gel or as a formulation capable of sol-gel transition in physiological conditions. Preferably, the premixed composition is injectable. Solid gels are not excluded from such preferred embodiments if the gel is, for example, a thixotropic gel, i.e. a gel exhibiting progressive decrease in viscosity with time under constant applied shear stress, such as agitation, followed by a gradual recovery when the stress is removed. As an alternative to being in aqueous form, the premixture may be provided in dry, preferably lyophilized form, e.g. as a powder, a cake or the like, that is to be reconstituted on demand with a physiologically acceptable solution, preferably for administration by injection, more preferably by intradermal or subcutaneous injection.

In an embodiment, provided is a device in which the cell-free lipoaspirate-derived preparation and the at least one biodegradable crosslinked hydrogel are provided in different compartments of a single container, such as a vial, an ampoule, a bottle, a syringe, a cartridge or the like. In such instances, the lipoaspirate-derived component and the hydrogel component may be provided, independently from each other, either in aqueous or in dry form as described above. If in dry form, the component in question is to be reconstituted into an aqueous form before use. This may be carried out by dissolving the dry component(s) in an appropriate physiologically acceptable solution or, if one component is in dry form and the other component is in aqueous form, by dissolving the dry component in the aqueous component. In any case, the components are to be combined and thoroughly mixed together to obtain a combination product for administration to a human body, preferably by injection, more preferably by intradermal or subcutaneous injection.

In an embodiment, provided is a multi-part device in which the cell-free lipoaspirate-derived preparation and the at least one biodegradable crosslinked hydrogel are provided in different containers. The containers may be of the same or different type, including without limitation vials, ampoules, bottles, syringes, cartridges and the like. Again, the lipoaspirate-derived component and the hydrogel component may be provided, independently from each other, either in aqueous or in dry form as described above. If in dry form, the component in question is to be reconstituted into an aqueous form before use. This may be carried out by dissolving the dry component(s) in an appropriate physiologically acceptable solution or, if one component is in dry form and the other component is in aqueous form, by dissolving the dry component in the aqueous component. In any case, the components are to be combined and thoroughly mixed together prior to administration to a human body, preferably by injection, as a combination product.

In an embodiment of the multi-part device, the cell-free lipoaspirate-derived preparation is provided in a first a syringe or a cartridge configured for coupling with a syringe body, and the biodegradable crosslinked hydrogel is provided in a second syringe or a cartridge configured for coupling with a syringe body. In such instances the device may further include a connector configured for connecting the first and the second syringes or syringe bodies together such that repeated alternating pressing of the plunger of the first syringe and the plunger of the second syringe allows thorough mixing of the cell-free lipoaspirate-derived preparation and the biodegradable crosslinked hydrogel.

In an embodiment, the connector comprises a body, with a core that is an open channel to allow movement of fluid-like materials to flow between at least two open ports in the body, each port comprising a Luer Lock fitting. The Luer Lock fittings are preferably of the same kind (either female/female or male/male), configured for leak-free coupling to syringes with Luer Lock fittings that are opposite and complementary to the port fittings, and for allowing connection between a first syringe and a second syringe, or the like.

In an embodiment, the connector has a body with at least two ports for connecting with two syringes or the like, with the connector preferably having a cylindrical body with a two-way open channel connecting the ports, more preferably a channel with constricted path to enhance shear and mixing, and most preferably a constricted path with a complex inner geometry, such as spiral, screw-like, grid-like, or plate-like, channel design to enhance the interfacial surface area and mixing of the composition.

Suitable commercially available connectors (also known as adaptors) may include, but are not limited to, for example B.Braun Combifix^{®} (female/female or male/male), Baxter Rapid-Fill^{™} (female/female) and Nobamedi Syringe mixing tube (MT-SEM01) adaptors. Further, accessories known to those skilled in the art, for example BD Connecta^{™} 3-way Stopcock, can be used for syringe-to-syringe mixing. By adjusting the position of the 3-way stopcock, the channel dimensions can be modified to create a flow path to have either greater or lesser degree of constriction. Specifically, when applying adaptors for syringe-to-syringe mixing, purging of air (displacing with the mixing compositions) from the system is required and is standard practice to those skilled in the art to avoid creating bubbles during mixing.

Containers suitable for the present devices are generally such that they are compatible with the present composition, i.e. do not harm its integrity or biological activity, and do not release particles or other substances beyond a physiologically acceptable limit into the composition contained therein. Those skilled in the art can easily select appropriate materials meeting these requirements, for example among glass, metals and plastics. In an embodiment, the material is cyclic olefin polymer (COP) or cyclic olefin copolymer (COC), preferably silicon oil-free COP or silicon oil-free COC. In another embodiment the material is silicon-oil free glass. These materials can be available in silicon-oil free format due to suitably coated stoppers (e.g. fluoropolymer coating on stopper) that provide suitable break loose and gliding forces to mitigate the lack of silicone coating or lubrication that is typically used. Moreover, only sealable containers equipped with stoppers, caps, lids or other appropriate closure strategies known to those skilled in the art, should be used in the present devices for preventing oxygen ingress as well as for protecting the container's contents in other respects.

Any of the devices of the present invention may be provided in a kit including not only one or more devices of the invention but also accessories such as accessories for reconstitution, mixing and administration depending on the particulars of the case, such as format of the composition and/or the device, mode of intended administration, type of intended treatment, and indication to be treated. Those skilled in the art can easily select appropriate accessories depending on these and other possible particulars. For example, for hypodermal administration, the kit may comprise one or more hypodermic needles and/or cannulae, preferably with gauges in the range of 25G to 32G and 22G to 27G respectively. Accessories for reconstitution may include either physical equipment for carrying out the reconstitution, or one or more physiologically acceptable aqueous reconstitution solutions, or both.

In an embodiment, the kit comprises one or more devices of the invention wherein at least one component of the present composition contained in the device is in dry, preferably lyophilized form, and a physiologically acceptable aqueous reconstitution solution selected from the group including, but not limited to, sterile water for injection, saline, phosphate buffer, and ringer acetate.

The hydrogel component provides not only instant volumetric augmentation, including both filling of wrinkles and smooth lines and restoration of volume at the site of administration, but also provides extended release of the lipoaspirate-derived component. Extended release is beneficial over immediate release since it prolongs the time the site of administration is exposed to the bioactive agents of the lipoaspirate-derived component, thereby improving its biostimulatory effects. Without being limited to any theory, the lipoaspirate-derived component enables long-term maintenance of the volume initially created by the hydrogel component, through natural subcutaneous tissue growth that compensates for the gradual degradation of the hydrogel component.

Owing to various bioactive agents present in the lipoaspirate-derived component, the present composition and combination product can provide aesthetic effects beyond an immediate filling or volumising effect furnished by the hydrogel component. Accordingly, the present composition and the combination product may be denoted as a biostimulatory dermal filler to distinguish it from conventional dermal fillers as well as from known biostimulatory non-filler injections. The lipoaspirate-derived component is also capable of not only contributing to the general well-being of the skin but also promoting skin regeneration, such as renewal of epidermal and dermal cells, especially activation of keratinocytes and fibroblasts, respectively.

The lipoaspirate-derived component activates microvascularization in the implant-tissue interface, with further induction of local cells. This lipoaspirate-derived component is capable of attracting and inducing fibroblasts, cells that produce ECM components to the site of administration, adipose stem cells that have the ability to differentiate into different skin and subcutaneous tissue cells, as well as keratinocytes, a type of epidermal cells, in the upper layers of skin. Overall, the activation of microvascularisation and the induction of local cells enables new natural-like soft tissue formation.

As used herein, the term "skin regeneration" refers to two processes which can occur in all skin layers. Firstly, to continuous skin renewal where skin cells and ECM are replaced with new cells and ECM *via* turn over process, and secondly to process where damaged tissue is replaced with new healthy tissue which is for example the final step in wound healing. Skin regeneration after wounding happens in humans only during embryonic development. In adults wound healing ends in fibroproliferative process, where a scar is formed to the site of an injury. Aging slows down the skin regeneration, which results in accumulation of skin defects and results in lower skin quality which is seen as a less elastic, thinner and more wrinkled or textured skin.

Without being limited to any theory, the lipoaspirate-derived preparation in the present composition and combination product may promote skin regeneration, thereby also enabling management of scars and other skin defects, as well as diminishing visible signs of skin aging, by a number of different mechanisms of action. For example, the preparation not only brings essential structural components (i.e., building blocks) and nutrients to the skin but also induces the local cells to produce more essential factors. Moreover, as demonstrated in the examples, the preparation is capable of activating migration of fibroblasts. Increased fibroblast migration is commonly known to play a role in wound healing and skin regeneration processes, thus the obtained results support cell-free lipoaspirate-derived preparation's ability to induce skin regeneration and wound healing by attracting fibroblasts to the site of administration. Fibroblasts can produce ECM components like collagen which is important as a structural scaffold to support new tissue formation and to provide structural support to the skin tissues. Fibroblasts are dispersed throughout the human body, including the dermis and subcutaneous tissues. Further results indicate that lipoaspirate-derived preparation is able to induce the production of ECM components in fibroblasts. Further results indicate that the present lipoaspirate-derived preparation is capable of activating keratinocytes and enhancing their turnover, thereby improving the barrier function of the skin upon intradermal administration of the present combination product.

It is to be noted that even when the combination product is applied to a specific layer of the skin, it can also produce beneficial effects on adjacent layers. For example, when administered to the subcutaneous layer, positive effects may extend to both the dermis and epidermis. Similarly, application to the dermis can benefit both the epidermis and subcutaneous layers. This layered interaction underscores the product's ability to enhance overall skin health and quality through its multi-layer influence.

Accordingly, in an embodiment, the present composition or the combination product is a cosmetic product intended for skin regeneration and/or skin rejuvenation, especially at the facial and/or neck area of a human body. In a further embodiment, the present composition or the combination product is a cosmetic product intended for improving the appearance of scars and/or reversing scarring. Preferably, these cosmetic products are injectable products, especially products intended for intradermal or subcutaneous administration.

In an embodiment, the combination product is intended for filling of superficial wrinkles or fine lines, in addition to promoting skin regeneration and general wellbeing of the skin, especially aged skin. In an embodiment, said product is intended for intrahypodermal administration. In an embodiment, said product is intended for intradermal administration

In an embodiment, the combination product is intended for aesthetic treatment of soft tissue loss. Advantageously, an immediate primary effect of volume augmentation, including volumisation at sites of subcutaneous volume loss, and long-term volume maintenance through natural subcutaneous tissue growth are achieved. In an embodiment, said product is intended for subcutaneous administration.

Accordingly, in an embodiment, the present combination product is more generally intended for regenerative aesthetics including skin regeneration and/or short and long-term volume augmentation.

In an embodiment, the present composition or the combination product is a cosmetic product, preferably a biostimulatory dermal filler, more specifically a facial and/or neck filler with a biostimulatory activity, and preferably with a long-term volumising effect. Preferably, the cosmetic product is an injectable product, especially a product intended for intradermal or subcutaneous administration.

Aging skin becomes drier and thinner, loses its firmness and appears more wrinkled. The moisture content decreases in the outermost layer of the skin, which is partly due to the reduction of lipids, i.e. fatty substances, and partly due to decrease in hyaluronic acid. Both of these changes affect the skin such that it is no longer able to bind and retain water in the same way as a younger skin does. The cell-free preparation of the invention contains a wide number of different lipids, fatty acids, associated proteins, and proteins related to lipid metabolism, which are considered to help the skin retain moisture thereby reducing its dryness, and thus not only rejuvenating aged skin but also providing means to manage defects that benefit from reduced dryness of the skin.

To be more specific, based on untargeted compositional analysis of the lipoaspirate-derived preparation, it essentially contains lipids, associated proteins and proteins related to lipid metabolism such as but not limited to ceramides and sphingolipids, apolipoproteins, perilipins, fatty acids, lysophospholipids, phospholipids, sphingomyelins, and di- and triacylglycerols. Some of these molecules may originate from the lipid fraction of a lipoaspirate, while some other molecules may originate from the aqueous fraction of a lipoaspirate. Accordingly, some of the components in the lipoaspirate-derived preparation may be fat-soluble while some other components may be water-soluble or partly water-soluble. Ceramides and sphingolipids are particularly interesting components of the preparation according to the invention because they are lipids found in normal skin cells.

In accordance with the other proposed mechanisms of action, characterization of the lipoaspirate-derived component also revealed that it essentially contains also structural and non-structural ECM proteins as well as peptides, chains, and/or subunits of said ECM proteins, as well as proteins related to the synthesis of extracellular matrix. Examples of said ECM proteins include, but are not limited to, Collagens I, III, IV, VI, XV, XVIII, fibronectin, vitronectin, elastin, hyaluronan, decorin, tenascin, laminin, lumican and prolargin. Lumican is a proteoglycan that induces fibrillogenesis of collagen, whereas prolargin is a protein that anchors the basement membrane to the underlying tissue.

Moreover, the lipoaspirate-derived component may contain basement membrane (BM) proteins as well as peptides, chains, and/or subunits of said BM proteins, as well as specialized dermoepidermal junction components. Examples of said BM and dermoepidermal junction proteins in the lipoaspirate-derived component include collagen, keratin, fibronectin, nidogen, laminin, versican, and intergrin linked proteins.

It is to be understood that although the lipoaspirate-derived component may contain components of the ECM and the BM, it contains neither intact ECM nor intact BM and is thus fundamentally different from any decellularized compositions. In this context, intact ECM and/or intact BM refers to compositions, in which the three-dimensional structure and composition of the ECM and/or BM are preserved, respectively. The preparation does not contain distinct large components of the ECM and/or BM either. As an example, the preparation does not contain intact collagen fibrils, but may contain different collagen molecules, microfibrils or subunits. Collagen exists in different forms, including at least collagens I, III, IV, VIII, and in different molecular sizes. The method for producing the lipoaspirate-derived component does not involve extraction of collagens or other large ECM or BM components, and does not even enable the presence of such intact components in the resulting preparation.

It can be understood that the lipoaspirate-derived component has a composition that is mimicking natural extracellular fluid found in the skin. Accordingly, combining the lipoaspirate-derived component with the hydrogel component, this property is imbued to the combination product and enhances the biocompatibility of the product. It creates a "biomimetic" interface between the product and the surrounding tissue. This interface provides sites that promote cell attachment and tissue integration while not promoting excessive fibrosis or inflammatory response.

Moreover, in those embodiments, which involve filtering through a 0.2 µm or a 0.22 µm filter, no components larger in size than said pore size can be present. It is to be noted that intact collagen I fibrils, for example, can be significantly larger in size than the pore size of a 0.2 µm filter or a 0.22 µm filter. Thus, although the filtered preparation may contain various peptides, chains and microfibrils of collagens and other ECM or BM components, their molecular size is limited to 200 nm or 220 nm, depending on the pore size of the filter.

Notably, the lipoaspirate derived component also contains essential and non-essential amino acids, antioxidants, oxidoreductases, vitamins as well as derivatives and metabolites of said vitamins, and a wide number of glycoproteins and proteoglycans as well as factors related to cell proliferation. These findings are also in accordance with the proposed mechanisms of action, although the present invention is not limited to any theory or mechanism of action. Tretinoin, a vitamin A derivative, is a particularly interesting bioactive agent in the lipoaspirate-derived component because it is an antioxidant that is famous for its anti-aging benefits and therefore often added into various skin care products. Tretinoin may also be classified into vitamins, more specifically vitamin derivatives or metabolites.

Also provided herein is use of the present composition and the combination product as a cosmetic product, preferably intended for one or more aesthetic, i.e., non-therapeutic, purposes including, but not limited to, filling of superficial tissue defects such as wrinkles and smooth lines; volume augmentation for deeper volume deficits, such as those in the facial and/or in the neck area of a human body, including volumisation provided by the hydrogel component and/or soft tissue regeneration provided by the lipoaspirate-derived component; skin regeneration; skin rejuvenation; improving the appearance of skin defects such as scars, including enhanced fading of the scar colour; and reversing scarring to restore healthy appearance of the skin. The volume augmentation may include contouring of the chin or any other facial or neck area of the human body.

In an embodiment the cosmetic product is an injectable product, especially a product intended for intradermal or subcutaneous administration.

Also provided is a non-therapeutic method for any of the aesthetic purposes disclosed herein, the method comprising: administering the present composition or the combination product to a selected area of the human body, typically to the facial area such as cheeks or the chin and/or to the neck area, preferably in a cosmetically efficient amount.

As used herein, the expressions "aesthetic treatment", "cosmetic treatment" and "non-therapeutic treatment" may be used interchangeably, all referring to the administration of the present composition or combination product to a subject in need thereof for a purpose which may include one or more of the aesthetic purposes disclosed in the preceding paragraphs.

As used herein, the term "cosmetically efficient amount" refers to an amount by which signs of cosmetic defects, such as superficial wrinkles or smooth lines, volume loss, or unsightly scars, are at least reduced or ameliorated.

It is to be understood that embodiments described in the preceding paragraphs may be used in any combination with each other. Several of the embodiments may be combined together to form a further embodiment.

In addition, any feature disclosed in the context of a more generally disclosed embodiment may apply, unless explicitly indicated otherwise, to a corresponding more specific embodiment even if the description of said feature is not repeated in relation to the more specific embodiment. For example, any feature disclosed in relation to crosslinked hydrogels in general, applies also to crosslinked hyaluronic acid. Moreover, any embodiment or other feature disclosed in the context of an aspect of the present invention may also apply to another aspect of the present invention, unless explicitly indicated otherwise.

It is also to be understood that a singular noun, unless otherwise specified, carries also the meaning of the corresponding plural noun. In other words, the singular expressions "a", "an" and "the" carry not only the meaning of "one" but also "one or more", unless otherwise specified.

Moreover, the term "and/or" in a phase such as "X and/or Y" shall be understood to mean either "X and Y" or "X or Y" and shall be taken to provide explicit support for both meanings or for either meaning.

Furthermore, the terms "comprising", "including", "containing" and "having" can be used interchangeably, and are intended to be construed in a non-exclusive manner, namely allowing for items, components or elements not explicitly described also to be present.

It is also to be understood that the embodiments described hereinbefore may be used in any combination with each other. Accordingly, two or more of the embodiments may be combined together to form a further embodiment. Furthermore, a composition, combination product, device or kit may comprise at least one of the embodiments described hereinbefore

### EXAMPLES

### Example 1

### Preparation of the cell-free lipoaspirate-derived preparation

Human lipoaspirate samples were obtained with signed informed consent from healthy volunteers undergoing standard water-assisted liposuction. The lipoaspirates were processed by allowing the lipoaspirates to separate into three layers; a lipid fraction on the top, an adipose tissue fraction containing adipocytes and other cells in the middle, and an aqueous fraction containing the used liposuction solution at the bottom. The aqueous fraction was collected. The cell-free preparations so obtained were stored at -20 degrees Celsius.

### Example 2

### Rheological properties after mixing the composition with different batches of the cell-free lipoaspirate-derived preparation to form a combination product

This and following Examples 3 to 7 were carried out for the purpose of demonstrating the mixability of the composition and resultant rheological properties of the combination product with different commercial crosslinked hyaluronic hydrogels that represent a wide spectrum of crosslinked hyaluronic acid formats, technologies and physiochemical properties, by mixing them with a cell-free lipoaspirate-derived preparation (CFLP) or an aqueous model ringer acetate (RA) solution to form a combination product. In all examples where CFLP is used, CFLP is brought to room temperature (+20-25 degrees Celsius) prior mixing or handling.

For Example 2, a commercial hyaluronic acid hydrogel filler indicated for subcutaneous injection in the facial area, Belotero^{®} Volume without lidocaine (Merz Aesthetics, REF: 11812), was mixed with different batches of CFLP prepared as described in Example 1 and RA (which served as a model simulating the CFLP for subsequent rheological examples).

Mixtures were prepared by syringe-to-syringe mixing of a single batch of the hydrogel (HA) with different batches of CFLP in liquid format containing different protein concentrations, 6655 ug/ml, 2130 ug/ml, 1959 ug/ml, and 0 ug/ml (RA solution only). Mixing was performed with the composition of a 50:50 (+/-1%) HA:CFLP vol:vol ratio using 30 back-and-forth (push-pull technique) manual mixing cycles. In all examples, unless otherwise specified; mixing was performed using two 1 ml plastic luer lock syringes (Chirana, REF: CH03001LL) connected with a syringe-to-syringe 2-way female/female luer lock adaptor (Baxter Rapidfill^{™} connector Luer Lock-to-Luer lock, REF: H93813901), mixing was always performed by the same person approximately 10-30 minutes before the rheological analysis, an undiluted unmixed control of the same HA was included in the analysis, and 0.25 ml of each sample was injected on the rheometer using a 27G hypodermic needle for each measurement.

The rheological properties, specifically the storage modulus (G'), were determined in oscillatory frequency sweeps on a TA Instruments Discovery Hybrid Rheometer-2 (DHR-2), equipped with parallel plate geometries (upper plate geometry with a diameter of 20 mm) and a measurement gap of 800 µm, a rest time of 60 seconds, at a frequency range of 0.1 to 100 Hz with a shear strain of 1 % in the linear viscoelastic region of the gels. The rheological values indicated are at 5 Hz. All measurements were performed at +25 degrees Celsius. Each sample was measured from the front (nozzle side), middle and back (plunger side) of the syringe to determine mixing homogeneity (standard deviation or stdev) and average values are reported. The results are summarized in TABLE 1, showing that the dilution reduced the G' of all the mixtures by approximately 40 %. This was consistent for the different batches of the cell-free lipoaspirate-derived preparation, and this was corresponding to that of the RA solution. The mixability of the composition and resultant homogeneity of the combination product was consistent across the whole syringe compared to the undiluted control. RA was used for all subsequent rheological experiments as a model simulating the CFLP.

**Table 1.**

| Sample description (protein concentration) | Ave. G'_{5HZ} (Pa) | Stdev (Pa) |
|---|---|---|
| Belotero^{®} Volume undiluted control (0 ug/ml) | 249.04 | 32.69 |
| Belotero^{®} Volume:CFLP 50:50 (6655 ug/ml) | 96.03 | 11.51 |
| Belotero^{®} Volume:CFLP 50:50 (2130 ug/ml) | 98.41 | 16.50 |
| Belotero^{®} Volume:CFLP 50:50 (1959 ug/ml) | 108.90 | 0.50 |
| Belotero^{®} Volume:RA 50:50 (0 ug/ml) | 99.22 | 8.74 |

### Example 3

### Rheological properties of the combination product after mixing the composition with different dilution ratios

For the purpose of demonstrating the mixability of the composition and resultant rheological properties of the combination product, Belotero^{®} Volume without lidocaine (Merz Aesthetics, REF: 11812), was mixed with a ringer acetate solution.

Mixtures were prepared by syringe-to-syringe mixing of a single batch of the hydrogel (HA) with RA solution. Mixing was performed with dilution ratios of 70:30, 50:50, 30:70 (+/-1 %) ofHA:RA vol:vol simulating possible dilution ratios for the composition using 30 back-and-forth (push-pull technique) manual mixing cycles.

The rheological properties were determined as per Example 2. Each sample was measured from the front (nozzle side), middle and back (plunger side) of the syringe to determine mixing homogeneity (stdev) and average values are reported. The results are summarized in TABLE 2, demonstrating that the dilution series showed a stepwise reduction of the G'. The homogeneity or mixability was consistent across the whole syringe compared to the undiluted control.

**Table 2.**

| Sample description | Ave. G'_{5HZ} (Pa) | Stdev (Pa) |
|---|---|---|
| Belotero^{®} Volume undiluted control | 249.04 | 32.69 |
| Belotero^{®} Volume:RA 70:30 | 139.09 | 14.46 |
| Belotero^{®} Volume:RA 50:50 | 99.22 | 8.74 |
| Belotero^{®} Volume:RA 30:70 | 59.38 | 7.76 |

### Example 4

### Rheological properties of the combination product after mixing the composition with different mixing cycles and adaptors

For the purpose of demonstrating the mixability of the composition and resultant rheological properties of the combination product 1) Belotero^{®} Volume without lidocaine (Merz Aesthetics, REF: 11812), 2) Juvéderm^{®} Voluma with lidocaine (Allergan, REF: 96637JR), 3) Restylane^{®} with lidocaine (REF: 011526), were mixed with a ringer acetate solution.

Mixtures were prepared by syringe-to-syringe mixing of a single batch of the hydrogel (HA) with RA solution. Mixing was performed of the composition with 50:50 (+/-1 %) HA:RA vol:vol ratio using back-and-forth (push-pull technique) manual mixing cycles. Mixing was performed using two1 ml plastic luer lock syringes (Chirana, REF: CH03001LL) connected either with a syringe-to-syringe 2-way female/female luer lock adaptor (Baxter Rapidfill^{™} connector Luer Lock-to-Luer lock, REF: H93813901) or a 3-way stopcock connector (BD connecta^{™}, REF: 394600) with a constricted pathway (minimum internal diameter approximately 2.0 mm).

The rheological properties were determined as per Example 2. Each sample was measured from the front (nozzle side), middle and back (plunger side) of the syringe to determine mixing homogeneity (stdev) and average values are reported. The results are summarized in TABLE 3, demonstrating that different mixing cycles showed that high mixing cycles improved the homogeneity across the whole syringe and that higher shear from the 3-way adaptor (smaller channel) resulted in improved homogeneity compared the same number of mixing cycles with the 2-way adaptor. Surprisingly with Belotero^{®} Volume higher storage moduli were seen when mixing with fewer cycles through the 3-way adaptor compared to more cycles through the 2-way adaptor.

**Table 3.**

| Sample description | Ave. G'SHZ (Pa) | Stdev (Pa) |
|---|---|---|
| Belotero^{®} Volume undiluted control | 249.04 | 32.69 |
| Belotero^{®} Volume:RA 50:50, 50 cycles/2-way adaptor | 100.59 | 8.21 |
| Belotero^{®} Volume:RA 50:50, 30 cycles/2-way adaptor | 99.22 | 8.74 |
| Belotero^{®} Volume:RA 50:50, 20 cycles/2-way adaptor | 91.43 | 8.09 |
| Belotero^{®} Volume:RA 50:50, 10 cycles/2-way adaptor | 84.74 | 13.76 |
| Belotero^{®} Volume:RA 50:50, 10 cycles/3-way adaptor | 93.34 | 10.60 |
| Juvéderm^{®} Voluma undiluted control | 331.83 | 3.05 |
| Juvéderm^{®} Voluma:RA 50:50, 50 cycles/2-way adaptor | 190.72 | 3.08 |
| Juvéderm^{®} Voluma:RA 50:50, 10 cycles/3-way adaptor | 257.93 | 17.41 |
| Restylane^{®} undiluted control | 596.51 | 8.72 |
| Restylane^{®}:RA 50:50, 50 cycles/2-way adaptor | 46.49 | 18.78 |
| Restylane^{®}:RA 50:50, 10 cycles/3-way adaptor | 96.40 | 22.51 |

### Example 5

### Rheological properties of the combination product after mixing the composition with a large range of various commercial hyaluronic acid hydrogels

For the purpose of demonstrating the mixability of the composition and resultant rheological properties of the combination product, a large range of commercial hyaluronic acid hydrogel fillers including a diverse range of crosslinking technologies from the most prominent brands were mixed with a ringer acetate solution. These are shown in TABLE 4.

Mixtures were prepared as per Example 2 but using 50 mixing cycles.

The rheological properties were determined as per Example 2. Each sample was measured from the front (nozzle side), middle and back (plunger side) of the syringe to determine mixing homogeneity (stdev) and average values are reported. The results are summarized in TABLE 4. Briefly, it is shown that the different commercial hydrogels demonstrate the range of storage modulus and homogeneity of mixing that could be obtained for the present composition and further how other hydrogels could be mixed to obtain optimal properties for a particular indication for soft-tissue augmentation. Examples 2-3 above demonstrate how other variables such as dilution ratio, mixing cycles and adaptor aperture may further influence the resultant combination product before injection.

**Table 4.**

| Sample description | HA Product REF (manufacturer) | Ave. G'SHZ (Pa) | Stdev (Pa) |
|---|---|---|---|
| Belotero^{®} Volume undiluted control | 11812 (Merz Aesthetics) | 249.04 | 32.69 |
| Belotero^{®} Volume:RA 50:50 | 11812 (Merz Aesthetics) | 100.59 | 8.21 |
| Belotero^{®} Intense undiluted control | 11698 (Merz Aesthetics) | 150.41 | 7.75 |
| Belotero^{®} Intense:RA 50:50 | 11698 (Merz Aesthetics) | 60.54 | 4.68 |
| Teosyal^{®} RH4 undiluted control | 94001/03 (Teoxane) | 237.79 | 26.14 |
| Teosyal^{®} RH4:RA 50:50 | 94001/03 (Teoxane) | 145.05 | 24.67 |
| Teosyal^{®} Ultra Deep undiluted control | 910049/04 (Teoxane) | 248.82 | 14.77 |
| Teosyal^{®} Ultra Deep:RA 50:50 | 910049/04 (Teoxane) | 164.31 | 25.49 |
| Juvéderm^{®} Voluma undiluted control | 96637JR (Allergan) | 331.83 | 3.05 |
| Juvéderm^{®} Voluma:RA 50:50 | 96637JR (Allergan) | 195.35 | 8.32 |
| Juvéderm^{®} Volux undiluted control | 96655JR (Allergan) | 581.73 | 46.10 |
| Juvéderm^{®} Volux:RA 50:50 | 96655JR (Allergan) | 331.13 | 14.61 |
| Juvéderm^{®} Ultra Plus 3 undiluted control | 96666JR (Allergan) | 172.72 | 11.70 |
| Juvéderm^{®} Ultra Plus 3:RA 50:50 | 96666JR (Allergan) | 100.53 | 4.61 |
| Restylane^{®} undiluted control | 011526 (Galderma) | 596.51 | 8.72 |
| Restylane^{®}:RA 50:50 | 011526 (Galderma) | 46.49 | 18.78 |
| Restylane^{®} Lyft undiluted control | 011534 (Galderma) | 534.05 | 42.47 |
| Restylane^{®} Lyft:RA 50:50 | 011534 (Galderma) | 93.86 | 87.07 |
| Restylane^{®} Defyne undiluted control | 011730 (Galderma) | 298.43 | 39.87 |
| Restylane^{®} Defyne:RA 50:50 | 011730 (Galderma) | 149.27 | 3.02 |
| Restylane^{®} Volyme undiluted control | 012001 (Galderma) | 211.56 | 44.54 |
| Restylane^{®} Volyme:RA 50:50 | 012001 (Galderma) | 118.80 | 8.50 |

### Example 6

### Influence of storage time and temperature after mixing of the composition and resultant rheological properties of the combination product

For the purpose of demonstrating the effect of storage time and temperature after mixing of the composition on the resultant rheological properties of the combination product, 1) Belotero^{®} Volume without lidocaine (Merz Aesthetics, REF: 11812) and 2) Juvéderm^{®} Voluma with lidocaine (Allergan, REF: 96637JR) were mixed with a ringer acetate solution.

Mixtures were prepared as per Example 5. Rheological analyses were performed on the day of mixing (1A and 2A) and also one week later for Belotero^{®} Volume:RA 50:50 (1B, syringe stored at room temperature +20-22 degrees Celsius) and 1 day later for Juvéderm^{®} Voluma:RA 50:50 (2B, syringe stored at +4 degrees Celsius for 24-26 h).

The rheological properties were determined as per Example 2 with the exception that the frequency range of 0.1 to 25 Hz was studied. Each sample was measured once from the front (nozzle side) of the syringe to determine if the rheology is in the same order of magnitude and that any potential swelling effects on the rheological measurement could be detected. The results are summarized in FIGURE 1. Surprisingly the storage modulus was equivalent for the different hydrogels irrespective of measurement time and temperature. This flexibility can be advantageous in a clinical setting, as the mixture can either be prepared at the time of administration or it can be prepared in advance if needed or if it has already been prepared it could still be useable in the case that the treatment must be postponed suddenly.

### Example 7

### Cohesivity after mixing the composition to form the combination product with a large range of various commercial hyaluronic acid hydrogels

For the purpose of demonstrating the cohesivity properties of the combination product, a large range of commercial hyaluronic acid hydrogel fillers including a diverse range of crosslinking technologies from the most prominent brands were mixed with a ringer acetate solution and a saturated (3.82 g/100 ml) aqueous toluidine blue solution (Merck, CAS: 92-31-9, a stain which co-ordinates with hyaluronic acid). These are shown in TABLE 6.

Mixtures were prepared by syringe-to-syringe mixing of a single batch of the hydrogel (HA) with ringer acetate solution (RA) and toluidine blue solution (TB). Mixing was performed of the combination product with 50:45:5 (+/-1 %) HA:RA:TB vol:vol:vol ratio using back-and-forth (push-pull technique) manual mixing cycles. Minimally diluted 95:5 (+/-1 %) HA:TB vol:vol mixed (using same protocol as above) controls of the same HA were included in the analysis. Mixing was always performed by the same person approximately 10-30 minutes before analysis.

0.5 ml of the stained (with toluidine blue) hyaluronic acid gel specimens were extruded into a beaker of sterile water (300 ml), from a height of approximately 2cm and stirred at a constant rate of 160 rpm with a magnetic stirring bar, 25 mm long. Dispersion was evaluated using the Gavard-Sundaram Cohesivity Scale. Serial photographs were captured at 15, 70, and 95 seconds after extrusion. The cohesivity scale was used for calculation at 70 second mark. Each photograph was graded by 2 evaluators on a scale of: fully dispersed (1), partially dispersed (2), partially cohesive (4) and fully cohesive (5). The average of the two measurements were recorded.

All measurements were performed at +20-22 degrees Celsius. The results are summarized in TABLE 6. To conclude, the different commercial hydrogels demonstrate the range of cohesivity after mixing that could be obtained for the combination product and further how other hydrogels could be mixed to obtain the optimal cohesivity or dispersity properties for a particular indication for soft-tissue augmentation. Examples 3-4 above demonstrate how other variables such as dilution ratio, mixing cycles and adaptor aperture may further influence the resultant combination product before injection. The mixtures that were more cohesive also tended to show better mixing homogeneity as seen in Example 6 above. Cohesivity properties and the release rate of the CFLP from the combination product are directly related. It is expected that the mixtures that are more cohesive will have a delayed release compared to systems that disperse easily. These properties may be beneficial depending on the indication and desired outcome.

**Table 6.**

| Hyaluronic acid | HA Product REF (manufacturer) | Cohesivity grade | |
|---|---|---|---|
| | | 95:5 vol:vol (HA:TB) | 50:45:5 vol:vol:vol (HA:RA:TB) |
| Belotero^{®} Volume | 11812 (Merz Aesthetics) | 4 | 4 |
| Belotero^{®} Intense | 11698 (Merz Aesthetics) | 5 | 5 |
| Teosyal^{®} RH4 | 94001/03 (Teoxane) | 3.75 | 3.75 |
| Teosyal^{®} Ultra Deep | 910049/04 (Teoxane) | 2 | 1.75 |
| Juvéderm^{®} Voluma | 96637JR (Allergan) | 1 | 1 |
| Juvéderm^{®} Volux | 96655JR (Allergan) | 1 | 1 |
| Juvéderm^{®} Ultra Plus 3 | 96666JR (Allergan) | 2.5 | 1 |
| Restylane^{®} | 011526 (Galderma) | 1 | 1 |
| Restylane^{®} Lyft | 011534 (Galderma) | 1 | 1 |
| Restylane^{®} Defyne | 011730 (Galderma) | 3.75 | 3.5 |
| Restylane^{®} Volyme | 012001 (Galderma) | 3.75 | 3.75 |

### Example 8

### Protein release profiles

These experiments were carried out to see how rheological properties of hyaluronic acid (HA) hydrogels affect the protein release profiles of combination products containing HA hydrogel and CFLP. To determine at what rate the combination product implant releases the cell-free lipoaspirate-derived preparation, protein release profiles were determined from three different hyaluronic acid -based hydrogels. Belotero^{®} Volume (Merz Aesthetics, REF: 11812), Restylane^{®} Lyft, (Galderma, REF: 011534) and Juvéderm^{®} Voluma (Allergan, REF: 96637JR) were selected for the profiling.

The hydrogels were mixed with CFLP with a known protein concentration in approximately 1:1 ratio, with a total protein amount of approximately 450 µg. The hydrogels were mixed with CFLP utilizing two luer-lock syringes (Chirana, REF: CH03001LL) connected with a two-way adaptor (Baxter Rapidfill^{™} connector Luer Lock-to-Luer lock, REF: H93813901). The hydrogel and CFLP were mixed for a total of 50 cycles where one cycle means moving components from one syringe to another and back.

After mixing, 0.25 ml of the combination product implant was injected through size 27G injection needle to a cell culture insert with a pore size of 0.4 µm (Sarstedt, REF: 83.3932.040). Inserts containing prepared implants were placed in wells in a 24-wellplate (Sarstedt, REF: 83.3922) filled with 1 ml of 1x PBS (Gibco). The well plate was then sealed with parafilm and placed in a heating cabinet with a set temperature of +37 degrees Celsius until sample collection. Samples were collected in 24-hour intervals over 4 days (D1, D2, D3, D4) and at each timepoint all of the PBS was collected and replenished with fresh PBS and collected samples were stored in -20 degree Celsius until further analysis. Protein concentrations were measured from samples using Pierce BCA protein assay kit (Thermo Fisher Scientific, REF: 23227).

Results from the protein concentration measurements are presented in the Figure 2 (2A: Restylane^{®} Lyft:CFLP 1:1, 2B: Juvéderm^{®} Voluma:CFLP 1:1 and 2C: Belotero^{®} Volume:CFLP 1:1) demonstrating how less cohesive hydrogel Restylane^{®} Lyft has significantly higher burst release during the first 24 hours when compared to highly cohesive Belotero^{®} Volume (p-value on two sample t-test = 0.001344). Results also show higher standard deviation in measured protein concentrations in less cohesive hydrogels indicating poor mixing compatibility. Hydrogels with lower burst release support the claim for prolonged protein release for a longer time period. These results demonstrate that the release can be somewhat customized for a particular indication by appropriate selection of a hydrogel with specific physiochemical properties.

### Example 9

### Fibroblast migration

This experiment was carried out to demonstrate that bioactive substances of the cell-free lipoaspirate-derived preparation are released from the crosslinked hydrogel in biologically active form. Fibroblast migration was chosen as a model system for this purpose.

BJ fibroblasts (ATCC, CRL-2522) were used to study combination products effect on cell migration. BJ fibroblasts were cultured in MEM (Sigma) supplemented with 10 % FBS (Gibco), 1 % MEM non-essential amino acids solution (Gibco), 1 % antibiotic-antimycotic (Gibco) and 1 % l-glutamine (Sigma). For migration, 20 000 fibroblasts were cultured in 3-well cell culture-inserts (Ibidi^{®}, REF: 80369) until confluent. Cells were plated on day -1 and combination product was applied day 0. For this example, Belotero^{®} Volume (Merz Aesthetics, REF: 11812) was selected. Combination product was prepared as described in Example 8 with an addition of a vehicle control where CFLP was replaced with RA. Combination products were injected through a 27G size injection needle on a cell culture insert with a pore size of 0.4 µm (Sarstedt, REF: 83.3931.040). Before introducing implants to the cells, Ibidi^{®} cell culture inserts were removed, and cells were imaged with Leica DMi8 microscope. Area between cell populations created with Ibidi^{®} inserts was measured in ImageJ 1.54f using Labkit plugin. Cells were imaged again after 24 hours, and remaining cell free area was compared to the area size at the start of the experiment. Results (Figure 3) show that in combination product + CFLP treated cells (Figure 3, column B) area between cell populations is decreasing faster than in the vehicle RA control (Figure 3, column A) treated cells, indicating increased cell migration at 24 hours (p-value on two sample t-test = 0.00056).

### Example 10

### Human adipose stem cell differentiation

This experiment was carried out to demonstrate that bioactive substances of the cell-free lipoaspirate-derived preparation are released from the crosslinked hydrogel in biologically active form. Adipose stem cell differentiation towards mature adipocytes, i.e. triglyceride accumulation in cells, was chosen as a model system for this purpose. Human adipose stem cells (hASC) were used, and intracellular triglyceride accumulation was measured with AdipoRed^{™} (Lonza, PT-7009) assay. The cells were cultured in DMEM/F12 media supplemented with GlutaMax^{™}, 10 % human serum and 1 % antibiotic/antimycotic. hASCs were plated in a 24-well plate with a cell density of 20 000 cells/cm². The cells were plated on day -1 and the combination product was applied on day 0. The combination product was prepared as described in Example 7 along with a preparation of a vehicle control in which CFLP was replaced with RA. 0.25 ml of the combination product was injected through size 27G injection needle to cell culture insert with a pore size of 0.4 µm (Sarstedt, REF: 83.3932.040). Triglyceride accumulation in hASCs was inspected after six days in culture. Cells were imaged with Leica DMi8 microscope and fluorescence signal intensity was quantified with ImageJ 1.54f. Results (Figure 4) show that in combination product treated cells the intracellular triglyceride formation significantly increased (p-value on two sample t-test = 0.0259) Figure 4, column B) when compared to the vehicle control (Figure 4, column A). These results demonstrate that indeed the lipoaspirate-derived preparation is released from the combination product in a biologically active form.

### Example 11

### In vivo assessment of adipose tissue formation and volumisation effect in subcutaneous tissue

An *in vivo* rat study is conducted to assess local effects of an implanted combination product of hyaluronic acid (HA) and CFLP. The study is carried out according to the Finnish animal protection laws and under prior governmental authorization with appropriate ethical approvals.

In this study, three HA hydrogels representing distinct filler categories with diverse physicochemical properties are tested to see how they, in combination with CFLP, promote soft tissue formation in situ. The local effects of implantation are assessed from hematoxylin and eosin -stained histological images.

The study is conducted with approximately 300 g Sprague Dawley male rats. Combination product implants of approximately 100 µl are injected into rat dorsal subcutis, between the upper subcutis and the muscle layer. Plain HAs with RA are used as control implants, whereas normal rat subcutis and skin are used as negative controls. The effects are assessed at several timepoints (e.g. 0, 1, 4, 12 and 20 weeks) with three replicates. In the timepoints the animals are sacrificed, and the remaining implant and the surrounding tissue are cut for histological analysis. Histological samples are cut into approximately 1 mm² pieces and fixed in 4 % paraformaldehyde overnight, dehydrated in graded ethanol series and embedded in paraffin. The paraffin embedded samples are cut into 3 to 5 µm slices and stained with hematoxylin-eosin for histological analysis. The local effects of implantation e.g. adipose tissue formation, tissue (epidermis, dermis and subcutis) thickness, and local inflammatory cell count are assessed from H&E stained images and also from immunostained images when applicable.

The results demonstrate the combination product's potential in regenerative aesthetics, especially for providing and inducing subcutaneous volume augmentation and/or skin regeneration.

### Example 12

### Treatment of nasolabial folds

This example demonstrates the potential of the combination product to provide immediate and sustained volumisation for correction of nasolabial folds. Two nasolabial treatments were performed by the same plastic surgeon. The treatments were carried out using the combination (mixing ratio of 50:50 vol:vol of the CFLP prepared as described in Example 1 and Juvéderm^{®} Ultra 2 (HA)) on the one side of the face and a control of only the HA (no dilution) to the other side of the face. These were injected deep into the subcutaneous tissue of the nasolabial folds. Patient 1, a 52-year-old female with severe nasolabial folds (deep grooves), received 1 ml of the combination product into the left side of the face and 0.65 ml of the HA into the right side of the face. Patient 2, a 58-year-old female with severe nasolabial folds (deep grooves), received 1.05 ml of the combination product into the right side of the face and 0.7 ml of the HA into the left side of the face. Assessments of the effect were recorded at different follow-up intervals after the treatment by the plastic surgeon (for Patient 1) and a "blinded" assessment from another plastic surgeon (for Patient 2). The assessment results are summarized in TABLE 7. It was clear that the sides treated with the combination produced sustained volumisation in the nasolabial fold and there was improved skin quality with time compared to the sides treated with only HA. The treatment with the combination product also had no lumps compared to the control within the first days after treatment.

**Table 7.**

| **Interval (months)** | **Patient 1 Assessment** | |
|---|---|---|
| | **Combination product (1 ml)** | **HA control (0.65 ml)** |
| 0 (1-2 days) | Satisfied with volumisation result. Observation of no lumps. | Satisfied with volumisation result. Observation of lumps. |
| 2 | Patient healed well with no change in result. | Patient healed well with no change in result. Lumps disappeared. |
| 6 | Nasolabial fold is lower and skin appears softer, brighter and healthier compared to the control side. Similar results were observed by three independent observers. | Nasolabial fold is deeper compared to the treatment side. |
| 26 | No change to 6 months, nasolabial fold remained filled. | Volume is lost and nasolabial fold returned to the original condition. |

| **Interval (months)** | **Patient 2 Assessment** | |
|---|---|---|
| | **Combination product (1.05 ml)** | **HA control** (0.7 **ml)** |
| 0 (1-2 days) | Satisfied with volumisation result. Observation of no lumps. | Satisfied with volumisation result. Observation of lumps. |
| 2 | Patient healed well no change in result. | Patient healed well with no change in result. Lumps had disappeared. |
| 11 | Post-treatment volume has sustained. | Post-treatment volume has decreased. |
| 27* | Volume is preserved on the treatment side. | The control side has deteriorated such that it is worse than the pre-operation state. |

| | | |
|---|---|---|
| * Note: the patient had lost 5 kg weight resulting in overall decrease of facial fat | | |

### Example 13

### Treatment of mid-face volume loss (cheeks)

This example demonstrates the potential of the combination product to provide immediate and sustained volumisation for correction of volume loss in the cheeks and the benefit of having prolonged release of CFLP from HA implant. The treatment was carried out using the combination (mixing ratio of 3:2 vol:vol of the CFLP prepared as described in Example 1 and Belotero Volume (HA)) on the one side of the face and a control of only the CFLP (no dilution) to the other side of the face. These were injected into the subcutaneous tissue of the cheeks. Assessments of the effect were recorded at different follow-up intervals after the treatment. The patient, a 65 year old male with age-related volume loss, received 2.5 ml of the combination product into the right side of the face and 1.5 ml of the CLFP into the left side of the face. The treatment was performed and assessed by the same plastic surgeon 4.5 months after the treatment. It was reported that the side treated with the combination produced sustained volumisation in the cheek. On both sides the skin quality had improved, the skin tone was more even and the skin looked healthier.

## Claims

1. A composition comprising:
- a cell-free lipoaspirate-derived preparation comprising at least one of a lipid fraction and an aqueous fraction of a lipoaspirate; and
- at least one biodegradable crosslinked hydrogel.

2. The composition according to claim 1, wherein the aqueous fraction comprises spent liposuction solution, with or without a local anaesthetic and/or adrenaline.

3. The composition according to claim 1 or 2, wherein the lipoaspirate-derived preparation comprises the aqueous fraction of the lipoaspirate and the lipid fraction of the lipoaspirate as the only liposuction-derived material in the preparation.

4. The composition according to claim 1 or 2, wherein the lipoaspirate-derived preparation comprises the aqueous fraction of the lipoaspirate, the lipid fraction of the lipoaspirate, and a spent liposuction solution not collected as part of the lipoaspirate as the only liposuction-derived material in the preparation.

5. The composition according to any one of claims 1-4, wherein the hydrogel is selected from the group consisting of crosslinked hyaluronic acid-based hydrogels, crosslinked protein-based hydrogels crosslinked polyethylene glycol (PEG)-based hydrogels, crosslinked, polysaccharide-based hydrogels, crosslinked peptide hydrogels, and crosslinked thermosensitive hydrogels, wherein the hydrogel is preferably crosslinked hyaluronic acid-based hydrogel, more preferably crosslinked monophasic and/or cohesive hyaluronic acid-based hydrogel.

6. The composition according to any one of claims 1-5, wherein the hydrogel comprises a local anaesthetic and/or adrenaline.

7. A device or a kit for regenerative aesthetics, comprising the composition according to any one of claims 1-6.

8. The device or the kit according to claim 7, wherein the cell-free lipoaspirate-derived preparation and the at least one biodegradable crosslinked hydrogel are provided as a premixed composition in a single container.

9. The device or the kit according to claim 7, wherein the cell-free lipoaspirate-derived preparation and the at least one biodegradable crosslinked hydrogel are provided separately, preferably either in different compartments of a single container, or in different containers.

10. The device or the kit according to any one of claims 7-9, wherein at least one of the cell-free lipoaspirate derived composition and the biodegradable crosslinked hydrogel is provided in dry form, preferably as a lyophilised powder or cake.

11. The device or the kit according claim 9 or 10, wherein the cell-free lipoaspirate-derived preparation is provided in a first a syringe and the biodegradable crosslinked hydrogel is provided in a second syringe, the device or the kit further comprising a connector configured to connect the first and the second syringes together such that repeated alternating pressing of the plungers of the first and second syringes allows back-and-forth mixing of the cell-free lipoaspirate-derived preparation and the biodegradable crosslinked hydrogel.

12. The device or the kit according to any one of claims 7-11, further comprising one or more accessories for on reconstitution, mixing and/or administration

13. Use of the composition according to any one of claims 1-6 or the device or the kit according to any one of claims 7-12 for cosmetically treating a human body, preferably a facial and/or neck area of a human body.

14. The use according to claim 13, wherein the cosmetic treatment results in soft tissue augmentation.

15. A non-therapeutic method for cosmetically treating a human body, the method comprising the step of applying the composition according to any one of claims 1-6 or the device or the kit according to any one of claims 7-12 to a human body, preferably to a facial and/or neck area of a human body.
